Europäisches Patentamt

European Patent Office

Office européen des brevets

(11) Veröffentlichungsnummer: **0 143 412 B1**

## (12) EUROPÄISCHE PATENTSCHRIFT

(45) Veröffentlichungstag der Patentschrift: **19.02.92**

(21) Anmeldenummer: **84113942.1**

(22) Anmeldetag: **17.11.84**

(51) Int. Cl.⁵: **G01N 33/52**, G01N 33/538

(54) **Immunchemischer Schnelltest.**

(30) Priorität: **25.11.83 DE 3342627**

(43) Veröffentlichungstag der Anmeldung:
**05.06.85 Patentblatt 85/23**

(45) Bekanntmachung des Hinweises auf die
Patenterteilung:
**19.02.92 Patentblatt 92/08**

(84) Benannte Vertragsstaaten:
**AT BE CH DE FR GB IT LI LU NL SE**

(56) Entgegenhaltungen:
**EP-A- 0 021 214**
**EP-A- 0 073 593**
**DE-A- 3 217 032**
**FR-A- 2 379 072**

(73) Patentinhaber: **BOEHRINGER MANNHEIM GMBH**
**Sandhofer Strasse 116**
**W-6800 Mannheim 31(DE)**

(72) Erfinder: **Freitag, Helmut, Dr.**
**An der Ziegelhütte 13**
**W-6940 Weinheim(DE)**
Erfinder: **Rothe, Anselm, Dr.**
**Tiefenklingerweg 21**
**W-6943 Birkenau(DE)**

**Beschreibung**

Die vorliegende Erfindung betrifft immunchemische Schnellteste unter Verwendung chromatographischer Hilfsmittel und Verfahren zur immunchemischen Bestimmung geeigneter Proteine.

Immunchemische Teste gewinnen zur Bestimmung von Hormonen, Proteinen, Pharmazeutika usw. zum Beispiel in Blut-, Urin- und Stuhlproben, aber auch außerhalb der klinischen Chemie, etwa in der Lebensmittelanalytik oder bei der Analytik von Gewässerproben zunehmend an Bedeutung.

Der Vorteil dieser Teste liegt in der hohen Spezifität und Affinität der Immunreagenzien, die es erlaubt, auch geringe Mengen der nachzuweisenden Substrate neben höheren Konzentrationen von relativ ähnlichen anderen Verbindungen sicher zu bestimmen. Von Nachteil, insbesondere für Laien, sind die vielen Handhabungsschritte, die bei den herkömmlichen Testen viel Zeit beanspruchen und normalerweise nur mit umfangreichen Laborausrüstungen ausgeführt werden können. Für eine breitere Anwendung immunchemischer Bestimmungen ist es daher wünschenswert, spezifische Teste mit niederer Nachweisgrenze zur Verfügung zu haben, die ohne komplizierte Zusatzausrüstung und nach Möglichkeit ohne zusätzliche Vor- oder Nachbehandlung der Substrate einsetzbar sind und das Ergebnis in kurzer Zeit anzeigen.

Aufgabe der vorliegenden Erfindung war es deshalb, Schnellteste zu entwickeln, mit denen immunchemische Bestimmungen einfach und schnell auch für den Laien durchführbar werden.

Für immunchemische Teste sind beispielsweise folgende Reaktionsweisen bekannt:

1. Konkurrenzbindungsverfahren

Bei diesem Verfahren wird zu einer unbekannten Menge eines nachzuweisenden Antigens oder Haptens (im folgenden kurz als "Antigen" bezeichnet) eine genau bekannte Menge eines entsprechend markierten Antigens zugesetzt, die anschließend in Konkurrenz mit einem Unterschuß eines entsprechenden Antikörpers, der an einen festen Träger fixiert ist, zur Reaktion gebracht werden. Festphase und flüssige Phase werden getrennt und durch die Bestimmung der Menge des markierten Antigens in der festen oder flüssigen Phase auf die Menge des zu bestimmenden Antigens zurückgeschlossen.

2. IEMA-Prinzip

Hier wird das zu bestimmende Antigen mit einem markierten Antikörper im Überschuß zur Reaktion gebracht und in einem zweiten Schritt der nicht reagierte Antikörper an ein entsprechendes trägerfixiertes Antigen gebunden. Wiederum kann nach Trennung der Phasen durch Bestimmung der Markierung in der festen oder flüssigen Phase auf die Menge des Antigens geschlossen werden.

3. Sandwich-Prinzip

Bei diesem Verfahren wird ein bivalentes Antigen einerseits mit einem an eine feste Phase gebundenen Antikörper und andererseits mit einem markierten Antikörper umgesetzt und wiederum nach Trennung von flüssiger und fester Phase in eine der beiden Phasen die Konzentration des markierten Antikörpers bestimmt und damit auf die Konzentration des bivalenten Antigens geschlossen.

All diesen Verfahren gemeinsam ist es, daß einer der Reaktanten trägergebunden ist oder nachträglich gebunden wird, um eine Trennung zwischen Komplex und überschüssigem Reagenz zu ermöglichen. Davon abgesehen, daß diese Trägerfixierung zusätzliche Kosten verursacht und vor allem die immunchemischen Eigenschaften verändern kann, erfordern diese Verfahren, wie bereits oben gesagt, einen relativ hohen Aufwand bei ihrer Durchführung. Sie sind deshalb für einfache, billige Schnelltestverfahren nicht geeignet.

Aus DE-A-32 17 032 ist ein Verfahren und eine trockene chromatographische Säule für kompetitive immunologische Bestimmungen bekannt. Hierbei konkurriert ein Antikörper um das zu bestimmende Antigen und ein markiertes Antigen. Zur Durchführung dieses Testprinzips ist es deshalb notwendig, zwei aufwendig hergestellte Substanzen, nämlich Antikörper und markiertes Antigen, zur Verfügung zu stellen. Die Trennung von Antigen-Antikörperkomplex und Antigen erfolgt ausschließlich durch Unterschiede im Adsorptionsvermögen an den zur Chromatographie verwendeten Materialien.

EP-A-0 073 593 lehrt einen heterogenen kompetitiven Immunoassay mittels Ausschlußchromatographie, wobei Hapten und markiertes Hapten einerseits von Antigen-Antikörper-Komplexen andererseits getrennt werden. Das Chromatographiematerial muß hierbei als Suspension in einer Flüssigkeit eingesetzt werden und ist den Anforderungen zur Trennung der durch ihre Größenunterschiede charakterisierten Fraktionen angepaßt.

Für die erfindungsgemäßen Schnellteste sollten keine trägerfixierten Antikörper oder Antigene, sondern die bei der Immunisierung direkt anfallenden Antikörper gegebenenfalls in ihrer markierten Form eingesetzt werden. Unter Antikörper sollen erfindungsgemäß die vollständigen Antikörper, aber auch aktive Bruchstücke, beispielsweise Fab-Fragmente, verstanden werden. Antikörper können sowohl in polyklonaler, als auch monoklonaler Form verwendet werden. Überraschenderweise ist dies möglich, weil sich die Immunkomplexe aus Antikörper und Antigen aufgrund ihres unterschiedlichen Molekulargewichts bzw. ihrer unterschiedlichen Größe von nicht umgesetzten Antikörpern, bzw. Antigenen auf chromatographischem Wege trennen lassen und ein entsprechender Nachweis in den chromatographisch getrennten Fraktionen danach leicht möglich ist.

Methoden zur Trennung von Proteinen aufgrund ihrer verschiedenen Größe sind als Gelfiltration zum Beispiel an Sephadex®-, Sephacryl®- und Ultrogel® säulen wohl bekannt. Diese Methoden zeichnen sich aber durch besondere Ansprüche aus, so daß sie nur von angelernten Personen mit einer entsprechenden Laborausrüstung durchgeführt werden können. Das Gelmaterial muß zum Beispiel mehrere Tage vorgequollen werden, die Chromatographiesäulen müssen sorgfältig gefüllt, mit dem Lösungsmittel ins Gleichgewicht gebracht und ständig feucht gehalten werden, um eine gute Trennung zu erzielen. Darüber hinaus werden außer der Säule noch Vorratsbehälter für die Lösungsmittel, Pumpen, Fraktionssammler, Detektorsysteme etc. benötigt und darüber hinaus Probenvorbereitungs- und Chromatographiezeiten von mehreren Stunden benötigt, so daß diese Verfahren für eine Schnelltestuntersuchung nicht in Frage kommen.

Überraschenderweise wurde nun gefunden, daß man eine kapillaraktive Schicht, die aus einem trockenen, porösen, nicht quellendem, körnigem Material mit Molekularsiebeigenschaften (z.B. Porenglas, Kieselgel o.ä.) besteht, für eine rasche Trennung von Substraten entsprechender Molekülgröße verwenden kann, wenn man den Flüssigkeitstransport aufgrund der Kapillarkräfte ausnutzt. In Abhängigkeit vom Porendurchmesser chromatographieren die Substrate entsprechend ihrer Molekülgröße mehr oder weniger schnell, wobei hochmolekulare Substanzen, die aufgrund ihrer Größe nicht in die Poren eindringen können, unmittelbar hinter der Lösungsmittelfront chromatographieren. Es gelingt somit ohne vorherigen großen Aufwand, d.h. ohne diese Säulen vorher zu equilibrieren oder die Flüssigkeiten zusätzlich mit Pumpen zu bewegen, innerhalb von relativ kurzen Zeiten - 5 bis 30 Minuten bei Säulenlängen von 5 bis 20 cm - entsprechende Trennungen durchzuführen. Um ein "Schieflaufen" der Säule zu vermeiden, sollte der Durchmesser der Säule 0,5 - 5 mm betragen, vorzugsweise 1 - 2 mm. Da Immunkomplexe im allgemeinen wesentlich größer sind als die zu ihrer Bildung verwendeten Antikörper und Antigene, ist es möglich, bei Verwendung geeigneter Molekularsiebe den Porendurchmesser so zu wählen, daß die Immunkomplexe nicht oder nur wenig in die Poren eindringen, d.h. mit der Lösungsmittelfront oder kurz dahinter chromatographieren während die Antikörper in die Poren eindringen können und damit zurückgehalten werden bzw. wesentlich langsamer chromatographieren. Die vorgenannten trockenen Kapillarsäulen können deshalb für einen immunologischen Schnelltest eingesetzt werden.

Erfindungsgemäß sind damit zwei unterschiedliche Verfahrensweisen möglich:

1. Das antigenhaltige Substrat kann mit einem Überschuß des Antikörpers versetzt und vorinkubiert werden. Nach erfolgter Umsetzung wird dann die Mischung auf eine vorstehend beschriebene Molekularsiebsäule gegeben und entweder mit einem geeigneten Lösungsmittel, d.h. normalerweise mit einer wässrigen Pufferlösung oder mit einem Überschuß der Reaktionslösung chromatographiert. In der vom nicht umgesetzten Antikörper freien Lösungsmittelfront kann danach die Konzentration des Immunkomplexes nach einer der an sich bekannten Methoden bestimmt werden.

2. Für den Benutzer noch einfacher ist es, vorgefertigte Säulen zu verwenden, die in ihrem unteren Teil Molekularsiebmaterial enthalten, welches mit geeigneten Antikörpern imprägniert ist und im oberen Teil nicht imprägniertes Material zur Chromatographie enthalten. Wird der mit dem Antikörper imprägnierte Teil nunmehr mit antigenhaltigem Substrat beaufschlagt, bildet sich in der mobilen Phase der Immunkomplex aus, der aufgrund seiner Größe nicht in die Molekularsiebporen eindringen kann und somit schneller wandert als der nicht umgesetzte Antikörper, der in der stationären Phase in den Poren zurückbleibt.

Es erscheint außerordentlich überraschend, daß mit so einfachen Mitteln eine rasche und brauchbare Trennung möglich ist, da nach den Angaben der Literatur erwartet werden mußte, daß die trockene Säule durch die hindurchlaufende Flüssigkeit nur unvollkommen, d.h. unter Ausbildung von mehr oder weniger großen Lufteinschlüssen innerhalb und zwischen den Partikeln benetzt würde, wodurch eine Trennung unmöglich gemacht würde. Andererseits war anzunehmen, daß insbesondere bei der Methode 2 die Bildung großer Immunkomplexe, die nur außerhalb der Poren stattfinden kann, durch Diffusionsvorgänge so verlangsamt wird, so daß eine ausreichende Reaktionsgeschwindigkeit innerhalb der kurzen Kontaktzeit von einigen Sekunden nicht eintritt.

Die Erfindung ist in den Ansprüchen näher gekennzeichnet.

In einer bevorzugten Ausgestaltung der Erfindung wird eine Kapillare aus Glas oder Kunststoff mit einem Innendurchmesser von 0,5 bis 5 mm, vorzugsweise 1 bis 2 mm und einer Länge von 5 bis 20 cm, vorzugsweise 10 bis 20 cm an einem Ende mit einem saugfähigen, lockeren Material verschlossen. Auf dieses Verschlußmaterial wird eine 0,2 bis 10 mm dicke Schicht des mit einem markierten Antikörper getränkten Molekularsiebmaterials gegeben und die Säule mit weiterem nicht imprägniertem Molekularsieb gefüllt und am oberen Ende wiederum mit einem durchlässigen Verschlußmaterial abgedichtet. Wenn der markierte Antikörper nicht direkt detektiert werden kann, beispielsweise aufgrund seiner Farbe, Fluoreszenz oder Radioaktivität, kann der obere Teil der Säule zusätzlich noch eine Nachweiszone enhalten, in der beispielsweise entsprechende Reagenzien enthalten sind, um mit der Markierung des Antikörpers, beispielsweise einem angekoppelten Enzym, ein detektierbares Produkt zu erzeugen.

Beaufschlagt man eine solche Säule am unteren Ende mit einigen μl der zu untersuchenden Lösung und stellt sie anschließend in eine geeignete Entwicklungsflüssigkeit, dann wandert die Flüssigkeit bis zur vollständigen Bedeckung des Säuleninhaltes aufgrund der Kapillarkräfte nach oben. Bei Anwesenheit des Antigens kommt es, wie beschrieben, zur Komplexbildung und zu einer Separierung dieses Komplexes am oberen Ende der Kapillarsäule. Da die Chromatographie mit der vollständigen Befeuchtung automatisch beendet ist, ist eine besondere Überwachung seitens des Anwenders nicht notwendig. Das Resultat kann insbesondere bei einem qualitativen Test noch nach längerer Zeit abgelesen werden und ggf. sogar das Lösungsmittel verdampft und die Säule zu Dokumentationszwecken aufbewahrt werden.

In einer weiteren bevorzugten Ausführungsform wird das Molekularsiebmaterial in einer 0,05 bis 1 mm dicken Schicht ggf. unter Zusatz eines Bindemittels auf einem nicht saugfähigen Träger ausgebreitet und dieser in 5 bis 10 mm breite, 50 bis 150 mm lange Streifen unterteilt. Je nach Verwendungszweck ist das eine Ende dieser Streifen zusätzlich mit entsprechenden Antikörpern getränkt und dient zur Auftragung der Probe und das andere Ende ist zusätzlich mit Nachweisreagenzien für die Markierung des Antikörpers imprägniert und dient zum Nachweis der gebildeten Antikörper/Antigenkomplexe. Falls die drei Zonen getrennt aufgetragen werden, muß darauf geachtet werden, daß zwischen ihnen ein ausreichender kapillarer Kontakt besteht. Es ist jedoch auch möglich, zunächst die Beschichtungsmasse, die in einem geeigneten Lösungsmittel suspendiert ist, beispielsweise mit einer Rakel aufzustreichen und zu trocknen und anschließend die Reagenzien nachzuimprägnieren. Als undurchlässige Träger werden vorzugsweise Streifen aus Kunststoffen, beispielsweise Polycarbonat, Polyester, Polyamid oder Polyethylen verwendet, jedoch können auch Träger aus Glas vorteilhaft sein.

Wie bereits oben gesagt, sollen die für die Chromatographie verwendeten Molekularsiebe aus nicht quellendem Material bestehen. Es kommen deshalb vorzugsweise anorganische Träger und dabei insbesondere die verschiedenen im Handel befindlichen Typen von Porenglas oder Kieselgele mit definierter Porengröße in Frage. Die innere und äußere Oberfläche des anorganischen Trägermaterials kann jedoch mit einer geeigneten organischen Susbstanz, beispielsweise einem Polyol überzogen sein, die dieser die Adsorptionseigenschaften eines Agar- oder Cellulosegels verleiht und negative Einwirkungen des anorganischen Trägers auf die Antikörper, beziehungsweise Antigene verhindert. Da als Lösungsmittel für die Chromatographie vorwiegend wässrige Lösungen in Frage kommen, müssen die verwendeten Materialien genügend hydrophil sein. Falls dies nicht bereits durch die Herstellung der Materialien gewährleistet ist, hat es sich als vorteilhaft erwiesen, die Trägermaterialien vor der Verarbeitung in einer wässrigen, Netzmittel und geeignete Puffer enthaltenden Lösung zu suspendieren und die so imprägnierten Körnchen wiederum zu trocknen.

Als Netzmittel können alle bekannten anionischen, kationischen oder nicht ionogenen Netzmittel verwendet werden. Konzentrationen von 0,01 bis 0,1 % werden vorzugsweise angewendet, geringere Konzentrationen sind insbesondere bei von Natur aus hydrophilen Materialien ebenfalls brauchbar, höhere Konzentrationen können ebenfalls verwendet werden, solange sie die Immunkomplexe nicht beeinflußen. Als Netzmittel seien beispielhaft Glykoläther wie Polyoxyethylenstearat oder -laurat, Alkylsulfate wie Dodezylsulfat, Fettalkoholpolyglykoläther, Phenoläther, beispielsweise 10,5-Ethoxy-nonylphenol, Cetyltrimethylammoniumbromid und Laurinsäure-bis-2-hydroxyethylamin genannt.

Die übrigen Bestandteile der Imprägnierlösung entsprechen in etwa den in der späteren Entwicklungslösung enthaltenen Bestandteilen, d.h. Puffern, Salzen, Komplexbildnern, Sterilisationsmitteln etc., die als niedermolekulare Bestandteile langsamer chromatographieren als die Lösungsmittelfront, wodurch erreicht wird, daß auch an der Lösungsmittelfront stationäre Bedingungen herrschen.

Weiterhin hat es sich als vorteilhaft erwiesen, dem Chromatographiematerial zusätzlich eine möglichst feinpulvrige, inerte und ggf. hydrophobe Füllmasse beizumengen, wobei sich Talkum, hydrophobiertes Glas, Kieselgel und ähnliches besonders bewährt haben. Dieses Material vermindert einerseits das Ausschlußvolumen zwischen den Trägerpartikeln und führt damit zu einer besseren Trennung und verlangsamt andererseits die Laufgeschwindigkeit der flüssigen Phase, wodurch sich die Gleichgewichtseinstellung durch Verbesserung der Diffusion zwischen der mobilen und stationären Phase und damit wiederum die Trennleistung verbessert. Darüber hinaus wird die Füllung der Säule homogener, wodurch zusätzlich ein etwaiges Auftreten von Lufteinschlüssen vermieden wird.

Bei der Auswahl der Bindemittel zur Herstellung von beschichteten Trägern ist darauf zu achten, daß diese einerseits zu einer stabilen Beschichtung führen, andererseits aber die Laufeigenschaften des Trägermaterials nicht wesentlich verändern dürfen, d.h. insbesondere die Poren nicht zusetzen. Dies gelingt überraschenderweise dadurch, daß man eine wässrige Kunststoffdispersion beispielsweise Polyvinylpropionat oder Polyacrylat in Gemisch mit einem gut wasserlöslichen, bzw. quellbaren Bindemittel verwendet. Agar-Agar, Hydroxypropylzellulose und ähnliche hochmolekulare Materialien wurden mit Erfolg verwendet.

Obwohl es in vielen Fällen ausreicht festzustellen, ob das in Frage kommende Antigen in der Substratlösung enthalten ist oder nicht, ist es in den meisten Fällen erwünscht, eine halbquantitative oder quantitative Bestimmung durchzuführen. Soweit der Immunkomplex nicht von sich aus farbig ist, erweist es sich deshalb als notwendig, den Antikörper in irgendeiner detektierbaren Weise zu markieren.

Eines der ältesten Verfahren dieser Art ist die Markierung mit radioaktiven Isotopen, beispielsweise mit Jod (125) oder Tritium und Bestimmung der Menge der Radioaktivität der den Antikörper/Antigenkomplex enthaltenden Zone mittels eines Radioaktivitässcanners. Da das Hantieren mit radioaktiven Substanzen wiederum spezielle Laboratorien erfordert, ist es heutzutage vorzuziehen, die Markierung mit einem Fluoreszenzfarbstoff zu bewirken, so daß der Antikörper beziehungsweise der Immunkomplex an seiner Fluoreszenz bestimmt und ausgemessen werden kann.

Insbesondere bei Proteinen, die nur in geringer Konzentration vorliegen, wird der Antikörper vorzugsweise mit einem geeigneten Enzym gekoppelt und die Umsetzung geeigneter Substrate unter der katalytischen Wirkung dieses Enzyms für die Nachweisreaktion benutzt. Diese Auswertung kann, wie oben bereits beschrieben, entweder direkt auf dem Trägermaterial durchgeführt werden oder nach Abtrennung der den Komplex enthaltenden Zone und Suspendieren in einem geeigneten Lösungsmittel durch Photometrie in einer Küvette.

Eine halbquantitative Bestimmung ist darüber hinaus möglich, indem man der Endzone des Trägermaterials eine definierte Menge eines spezifischen Adsorptionsmittels für das Markierungsenzym und/oder den Komplex in trägerfixierter Form beimischt und aus der Breite der sich entwickelnden Absorptionszone auf die ursprüngliche Enzymmenge im Immunkomplex rückschließt.

In den folgenden Beispielen sind die erfindungsgemäßen Schnellteste näher ausgeführt.

## Beispiele

### Beispiel 1

Nachweis von hCG (human Chorionic Gonadotropin) im Urin mittels Testkapillare

### Lösungen

A. 3 mg Indolylgalactosid werden in 1 ml Methanol gelöst.

B. Fab-Anti-hCG-$\beta$-Galactosidase-Konjugat wurde aus Schaf-Anti-hCG-Serum hergestellt (Immunisierung: D.M. Weir, Handbook of Experimental Immunologie, A 2.8; Fab-Spaltung: M.E. Davis, A.J. Barrett, R.M. Hernbry, J. of Immunological Methods 21 305 (1978); Konjugation: T. Kitagawa in Enzyme Immuno Essay, Editors: E. Ishikawa, T. Kawai, K. Miyai, Verlag Igaku-Shoiu, Tokio - New York 1981) und zu 575 U/ml in 10 mM $KP_i$, 5 mM $MgCl_2$, 25 mM NaCl, 2 % Saccharose, 0,5 % Rinderserumalbumin, 0,1 % $NaN_3$, pH 7,0 gelöst.

### Materialien

(Porenglas: Glyceryl-Glas, Controlled Pore (Sigma, St. Louis)), 25,9 nm (259 Å) Porendurchmesser, Partikelgröße: 200 - 400 mesh.

Glaskapillare: Länge 12 cm, Innendurchmesser 1 mm, einseitig verstopft mit Zellstoff (2 mm Höhe).

### Bestücken der Kapillare

Die Kapillare wird mit etwas Zellstoff am unteren Ende abgedichtet und mit Porenglas gefüllt (Füllhöhe 7 cm). 500 mg Porenglas werden mit 1 ml Lösung A gemischt und anschließend bei 60 °C getrocknet. Die mit Porenglas bestückte Kapillare wird nun mit diesem Porenglas/Indolylgalactosid-Substrat-Gemisch (Füllhöhe 1 cm) überschichtet. Der Zellstoff am unteren Ende der Kapillare wird mit 3 $\mu$l Lösung B getränkt und getrocknet.

### Testdurchführung

Die Kapillare wird in den zu testenden Urin gestellt. Innerhalb von ca. 5 Minuten erreicht die Flüssigkeit die obere Substratschicht. Nur bei Anwesenheit von hCG erreicht die Galactosidase aus dem Immunkomplex diese Substratschicht. Das farblose Indolylgalactosid wird hydrolysiert und in Gegenwart von Sauerstoff zum Indigo umgesetzt, der visuell durch Blaufärbung erkennbar wird.

Beispiel 2

Nachweis von hCG im Urin mittels Teststreifen

Lösungen

A.    20 ml 0,3 % Agar-Agar in 150 mM NaCl,
10 mM $KP_i$, pH 7,2
+ 400 $\mu$l Triton® X-100
+ 2,3 ml Wasser
+ 2 g einer 50proz. Polyvinylpropionat-Dispersion in Wasser (Propiofan® 70 D, BASF, Ludwigshafen)

B.    Fab-Anti-hCG-$\beta$-Galactosidase-Konjugat aus Schaf-Anti-Serum wie in Beispiel 1.

Materialien

Kieselgel, (Daltosil®, SP 300, Porengröße ca. 30 nm (300 Å), Teilchengröße 120-200 mesh, Fa. Serva). Kieselgel-Indolylgalactosidgemisch (eine Lösung von 3 mg Indolylgalactosid in 1 ml Methanol wird mit 250 mg des vorstehenden Kieselgels versetzt und der erhaltene Brei bei 60 - 70 °C im Luftstrom getrocknet).
Polycarbonatfolie (Pokalon®, Fa. Lonza, Lörrach).

Beschichtung

Es werden folgende Mischungen hergestellt:
a) 8 ml Lösung A plus 1,5 g Kieselgel
b) 1 ml Lösung A plus 0,19 g Kieselgel-Indolylgalactosidgemisch
c) 0,15 ml Lösung A plus 0,05 ml Lösung B plus 0,04 g Kieselgel
Mit einer durch Trennwände (an einer Seite abgerundet) unterteilten Rakel werden die Mischungen a bis c in sich berührenden Schichten auf eine 15 cm breite Polycarbonatfolie ausgezogen (Schichtdicke 400 $\mu$m). Die Schichtbreiten und Reihenfolge sind dabei wie folgt:
Schicht a 1 cm, Schicht c 0,5 cm, Schicht a 8 cm, Schicht b 1 cm und überstehende Polycarbonatfolie als Handgriff 4,5 cm.
Die Folie wird bei 45 °C getrocknet und senkrecht zu der Beschichtung in 0,5 cm breite Teststreifen (Länge 6 cm) geschnitten.

Testdurchführung

Der Teststreifen wird mit dem unteren Ende in den Urin gestellt.

Beschreibung

Innerhalb von 6 Minuten hat die Flüssigkeit das oberste Ende des Teststreifens erreicht und die Anwesenheit von hCG führt wie unter Beispiel 1 zur Farbreaktion.

Beispiel 3

Einfluß von Netzmitteln auf die Trenneigenschaften von Porengläsern

Unbehandelte Porengläser zeigen schlechte Trenneigenschaften. Eine Vorbehandlung bringt erhebliche Verbesserungen. Die Gläser werden mit einer Lösung aus 0,025% Tween®20, 10 mM $Na_2HPO_4$, 25 mM NaCl, 0,01% $NaN_3$, pH 7,2 30 Minuten vorinkubiert, auf einer Nutsche abgesaugt und im Trockenschrank bei 35 °C bis zur Rieselfähigkeit getrocknet.
Als Maß für die Güte der Trennung wird das Einschlußvolumen mit einer niedermolekularen Substanz, Chlorphenolrot, in mit 11 cm langen, gefüllten Kapillarsäulen bestimmt. Porengläser: Firma Sigma, 120-200

6

mesh,
Auftrag: 4 µl Farblösung, Chromatographie in Wasser. Füllhöhe der Kapillarsäule: 11 cm.

| Porendurch-messer | unbehandeltes Material | | imprägniertes Material | |
|---|---|---|---|---|
| | Farbmaximum bei | Peak-breite | Farbmaximum bei | Peak-breite |
| 24 nm (240 Å) | 8,0 cm | 1,2 cm | 5 cm | 0,8 cm |
| 35 nm (350 Å) | 10,0 cm | 2,0 cm | 5 cm | 1,2 cm |
| 50 nm (500 Å) | 7,5 cm | 4,0 cm | 4,8 cm | 1,2 cm |
| 70 nm (700 Å) | 7,2 cm | 1,7 cm | 4,9 cm | 1,2 cm |
| 100 nm (1000 Å) | 10,3 cm | 1,3 cm | 4,8 cm | 0,8 cm |

Nach Angaben des Herstellers sollte man theoretisch, d. h. bei sorgfältig in Flüssigkeit aufgeschwemmtem und in Säulen equilibrierten Material, in diesem Beispiel 4,7 cm erreichen.

Beispiel 4

Einfluß von inerten Beimengungen auf die Trenneigenschaften von Porengläsern

Die Trenngüte bei der Gelchromatographie hängt auch von der Geschwindigkeit der Chromatographie ab. Eine zu schnelle Chromatographie führt zu verminderter Trennleistung. Die Geschwindigkeit der Chromatographie in mit trockenem Porenglas gefüllter Kapillarsäule kann durch Beimengung hydrophobierter Teilchen verringert werden. In der folgenden Tabelle ist der Einfluß der Beimengung von hydrophobiertem Kieselgel (Octyl-SP 500, 40 - 100 µm, Fa. Serva) auf die Laufgeschwindigkeit einer Kapillarsäule (1 mm Durchmesser, 10 cm Länge) mit Porenglas (Controlled Pore Glass, 24 nm (240 Å), 200 - 400 mesh, Fa. Serva) angegeben.

Tabelle

| % (W/W) Octyl-SP 500 | Chromatographiezeit Min. |
|---|---|
| 0 | 3,4 |
| 3 | 7,8 |
| 5 | 12 |
| 7 | 16 |
| 10 | 19 |
| 13 | 20 |
| 14 | 21 |
| 17 | 24 |
| 20 | 52 |
| 22 | 58 |
| 25 | 175 |

Beispiel 5

Trennung nach Molekulargewicht mit vorbehandelten Gläsern

7

CPG Porenglas, Fa. Serva, 200 - 400 mesh, Füllhöhe der Säulen: 10 cm, Auftrag: jeweils 3 $\mu$l einer Proteinlösung (2 mg Protein/ml), Peakbreite: 0,5 - 0,7 cm. Lösungsmittel: Wasser.

|  | Maximum bei | |
|---|---|---|
| Porendurchmesser | 24 nm (240 Å) | 35 nm (350 Å) |
| Cytochrom C MG = 12000 | 5,3 cm | 5,4 cm |
| Fluoresceinmarkierter Antikörper (Anti-Albumin) MG = 150000 | 6,5 cm | 5,6 cm |
| Ferritin MG = 450000 | 7,1 cm | 6,2 cm |
| Dextranblau MG ca. 6 Mill. | front | front |
| Fluoresceinmarkierter Antikörper (Anti-Albumin), Chromatographie in 1 mg Albumin/ml PBS | 1) 2) 6,5 + 8,1 cm | |

1) Antikörper, 2) Antikörper/Antigen-Komplex

## Patentansprüche

1. Verfahren zum Nachweis eines Antigens mittels einer immunchemischen Reaktion durch Komplexbildung von Antigenen mit Antikörpern, dadurch gekennzeichnet, daß man das antigenhaltige Substrat mit einem Überschuß des Antikörpers versetzt und die Mischung über eine Vorrichtung, bestehend aus einer kapillaraktiven Schicht aus einem trockenen, porösen, nicht quellenden, körnigen Material mit Molekularsiebeigenschaften, wobei die Porengröße des Molekularsiebes ein Eindringen der zum immunchemischen Nachweis der Antigene dienenden löslichen Antikörper ermöglicht, aber die aus Antigen und Antikörper gebildeten löslichen Immunkomplexe nicht oder nur wenig eindringen läßt, mit einem geeigneten Lösungsmittel chromatographiert und den gegebenenfalls gebildeten Immunkomplex im oberen Teil der Vorrichtung nachweist.

2. Verfahren zum Nachweis eines Antigens mittels einer immunchemischen Reaktion durch Komplexbildung von Antigenen mit Antikörpern, dadurch gekennzeichnet, daß man das antigenhaltige Substrat mit einem Überschuß des Antikörpers versetzt und die Mischung über eine Vorrichtung, bestehend aus einer kapillaraktiven Schicht aus einem trockenen, porösen, nicht quellenden, körnigen Material mit Molekularsiebeigenschaften, wobei die Porengröße des Molekularsiebes ein Eindringen der zum immunchemischen Nachweis der Antigene dienenden löslichen Antikörper ermöglicht, aber die aus Antigen und Antikörper gebildeten löslichen Immunkomplexe nicht oder nur wenig eindringen läßt, mit einem geeigneten Lösungsmittel chromatographiert und den gegebenenfalls gebildeten Immunkomplex im oberen Teil der Vorrichtung nachweist und wobei die Vorrichtung in ihrem unteren Teil mit geeigneten Antikörpern imprägniert ist oder geeignete Antikörper in einer vorgeschalteten porösen Schicht enthält.

3. Verfahren nach Anspruch 1 oder 2, dadurch gekennzeichnet, daß die antigenhaltige Substratlösung auch als Laufmittel für die Chromatographie dient.

4. Verfahren nach einem der Ansprüche 1 bis 3, dadurch gekennzeichnet, daß der Immunkomplex selbst oder ein damit gekoppeltes Markierungssystem nach der chromatographischen Abtrennung detektiert wird.

5. Vorrichtung zur Durchführung immunchemischer Schnellteste gemäß dem Verfahren nach einem der Ansprüche 1 bis 4 bestehend aus einer kapillaraktiven Schicht aus einem trockenen, porösen, nicht quellenden, körnigen Material mit Molekularsiebeigenschaften dadurch gekennzeichnet, daß die Porengröße des Molekularsiebes ein Eindringen der zum immunchemischen Nachweis von Antigenen dienenden löslichen Antikörper ermöglicht, aber die aus Antigen und Antikörper gebildeten löslichen Immunkomplexe nicht oder nur wenig eindringen läßt und der Antikörper auf den unteren Teil des Molekularsiebes imprägniert ist oder sich in einer vorgeschalteten porösen Schicht befindet.

6. Vorrichtung gemäß Anspruch 5, dadurch gekennzeichnet, daß die kapillaraktive Schicht aus einer Kapillarsäule mit einem Durchmesser von 0,5 - 5 mm und einer Länge von 5 - 20 cm besteht.

7. Vorrichtung gemäß Anspruch 5, dadurch gekennzeichnet, daß die kapillaraktive Schicht sich auf einem inerten Trägermaterial befindet und 0,05 - 0,5 mm dick, 5 - 10 mm breit und 5 - 20 cm lang ist.

8. Vorrichtung gemäß einem der Ansprüche 5 bis 7, dadurch gekennzeichnet, daß der Antikörper radioaktiv, fluoreszierend oder mit einem Chromophor markiert ist.

9. Vorrichtung gemäß einem der Ansprüche 5 bis 7, dadurch gekennzeichnet, daß der Antikörper mit einem Enzym markiert ist.

10. Vorrichtung gemäß Anspruch 9, dadurch gekennzeichnet, daß der obere Teil des Materials mit einem Reagenzsystem imprägniert ist, welches mit dem Markierungsenzym eine detektierbare Reaktion eingeht.

11. Vorrichtung gemäß einem der Ansprüche 5 bis 10, dadurch gekennzeichnet, daß dem Molekularsiebmaterial zusätzlich ein inertes, hydrophobes, feinkörniges Material beigemischt ist.

12. Vorrichtung gemäß einem der Ansprüche 5 bis 11, dadurch gekennzeichnet, daß das Molekularsiebmaterial hydrophil ist und/oder mit einem Netzmittel in einer Menge von 0,01 - 0,1 % imprägniert ist.

13. Vorrichtung gemäß einem der Ansprüche 5 bis 12, dadurch gekennzeichnet, daß das Molekularsiebmaterial zusätzlich mit Puffern und/oder Salzen und/oder Komplexbildnern imprägniert ist.

14. Verwendung trockenen, porösen, nicht quellenden, körnigen Materials mit Molekularsiebeigenschaften für den Nachweis eines Antigens durch Umsetzung mit einem Überschuß an Antikörpern und anschließende Trennung von freien Antikörpern und Antigen-Antikörperkomplexen.

**Claims**

1. Process for the detection of an antigen by means of an immunochemical reaction by complex formation of antigens with antibodies, characterised in that one mixes the antigen-containing substrate with an excess of the antibody and chromatographs the mixture with a suitable solvent over a device consisting of a capillary-active layer of a dry, porous, non-swelling, granular material with molecular sieve properties, whereby the pore size of the molecular sieve makes possible a penetration of the soluble antibody serving for the immunochemical detection of the antigen but does not permit or only permits slightly the penetration of the soluble immune complexes formed from antigen and antibody and

9

detects the possibly formed immune complex in the upper part of the device.

2. Process for the detection of an antigen by means of an immunochemical reaction by complex formation of antigens with antibodies, characterised in that one mixes the antigen-containing substrate with an excess of the antibody and chromatographs the mixture with a suitable solvent over a device consisting of a capillary-active layer of a dry, porous, non-swelling, granular material with molecular sieve properties, whereby the pore size of the molecular sieve permits a penetration of the soluble antibody serving for the immunochemical detection of the antigen but does not permit or only permits slightly the penetration of the soluble immune complex formed from antigen and antibody and detects the immune complex possibly formed in the upper part of the device and whereby the device is impregnated in its lower part with suitable antibodies or contains suitable antibodies in a superposed porous layer.

3. Process according to claim 1 or 2, characterised in that the antigen-containing substrate solution also serves as elution agent for the chromatography.

4. Process according to one of claims 1 to 3, characterised in that the immune complex itself or a labelling system coupled therewith is detected after the chromatographic separation.

5. Device for the carrying out of immunochemical rapid tests according to the process according to one of claims 1 to 4, consisting of a capillary-active layer of a dry, porous, non-swelling, granular material with molecular sieve properties, characterised in that the pore size of the molecular sieve makes possible a penetration of the soluble antibodies serving for the immunochemical detection of antigens but does not permit or only permits slightly the penetration of the soluble complexes formed from antigen and antibody and the antibody is impregnated in the lower part of the molecular sieve or is present in a superposed porous layer.

6. Device according to claim 5, characterised in that the capillary-active layer consists of a capillary column with a diameter of 0.5 - 5 mm and a length of 5 - 20 cm.

7. Device according to claim 5, characterised in that the capillary-active layer is present on an inert carrier material and is 0.05 - 0.5 mm thick, 5 - 10 mm wide and 5 - 20 cm long.

8. Device according to one of claims 5 to 7, characterised in that the antibody is labelled radioactively, fluorescingly or with a chromophore.

9. Device according to one of claims 5 to 7, characterised in that the antibody is labelled with an enzyme.

10. Device according to claim 9, characterised in that the upper part of the material is impregnated with a reagent system which enters into a detectable reaction with the labelling enzyme.

11. Device according to one of claims 5 to 10, characterised in that the molecular sieve material is additionally admixed with an inert, hydrophobic, fine-grained material.

12. Device according to one of claims 5 to 11, characterised in that the molecular sieve material is hydrophilic and/or is impregnated with a wetting agent in an amount of 0.01 - 0.1%.

13. Device according to one of claims 5 to 12, characterised in that the molecular sieve material is additionally impregnated with buffers and/or salts and/or complex formers.

14. Use of dry, porous, non-swelling, granular materials with molecular sieve properties for the detection of an antigen by reaction with an excess of antibodies and subsequent separation of free antibodies and antigen-antibody complexes.

**Revendications**

1. Procédé pour la détection d'un antigène au moyen d'une réaction immunochimique, par formation d'un complexe de l'antigène avec un anticorps, caractérisé en ce que l'on ajoute au substrat contenant l'antigène un excès d'anticorps et que l'on soumet le mélange à une chromatographie à travers un dispositif constitué d'une couche à activité capillaire et constitué d'un matériau sec, poreux, non gonflant, granulaire, ayant des propriétés de tamis moléculaire, la taille des pores du tamis moléculaire permettant une pénétration de l'anticorps soluble servant à la détection immunochimique de l'antigène, mais ne laissant pas, ou que peu, pénétrer l'immunocomplexe soluble formé à partir de l'antigène et de l'anticorps, à l'aide d'un solvant approprié, et l'on détecte l'immunocomplexe éventuellement formé dans la partie supérieure du dispositif.

2. Procédé pour la détection d'un antigène au moyen d'une réaction immunochimique, par formation d'un complexe de l'antigène avec l'anticorps, caractérisé en ce que l'on ajoute au substrat contenant l'antigène un excès d'anticorps et l'on soumet le mélange à une chromatographie sur un dispositif constitué par une couche à activité capillaire, consistant en un matériau sec, poreux, non gonflant, granulaire, ayant des propriétés de tamis moléculaire, la taille des pores du tamis moléculaire permettant une pénétration de l'antigène soluble servant à la détection immunochimique de l'anticorps mais ne permettant pas, ou que peu, la pénétration de l'immunocomplexe soluble formé à partir de l'antigène et de l'anticorps, à l'aide d'un solvant approprié, et l'on détecte l'immunocomplexe éventuellement formé dans la partie supérieure du dispositif, et dans lequel le dispositif est imprégné dans sa partie inférieure avec des anticorps appropriés ou contient des anticorps appropriés dans une couche poreuse montée en amont.

3. Procédé selon la revendication 1 ou 2, caractérisé en ce que la solution de substrat contenant l'antigène sert également d'éluant pour la chromatographie.

4. Procédé selon l'une quelconque des revendications 1 à 3, caractérisé en ce que l'immunocomplexe lui-même ou un système de marquage couplé à celui-ci est détecté après la séparation par chromatographie.

5. Dispositif pour la réalisation du test rapide immunochimique selon le procédé décrit dans l'une quelconque des revendications 1 à 4, constitué par une couche à activité capillaire formée d'un matériau sec, poreux, non gonflant, granulaire, ayant des propriétés de tamis moléculaire, caractérisé en ce que la taille des pores du tamis moléculaire permet une pénétration de l'anticorps soluble servant à la détection immunochimique de l'antigène mais ne permet pas, ou que peu, la pénétration de l'immunocomplexe soluble formé à partir de l'antigène et de l'anticorps, et que l'anticorps se trouve imprégné dans la partie inférieure du tamis moléculaire ou se trouve dans une couche poreuse montée en amont.

6. Dispositif selon la revendication 5, caractérisé en ce que la couche à activité capillaire est constituée par une colonne capillaire ayant un diamètre de 0,5 - 5 mm et une longueur de 5 - 20 cm.

7. Dispositif selon la revendication 5, caractérisé en ce que la couche à activité capillaire se trouve sur un matériau de support inerte et présente une épaisseur de 0,05 - 0,5 mm, une largeur de 5 - 10 mm et une longueur de 5 - 20 cm.

8. Dispositif selon l'une quelconque des revendications 5 à 7, caractérisé en ce que l'anticorps est marqué par radioactivité, par fluorescence ou à l'aide d'un chromophore.

9. Dispositif selon l'une quelconque des revendications 5 à 7, caractérisé en ce que l'anticorps est marqué au moyen d'une enzyme.

10. Dispositif selon la revendication 9, caractérisé en ce que la partie supérieure du matériau est imprégnée avec un système de réactifs qui entre en réaction détectable avec l'enzyme de marquage.

11. Dispositif selon l'une quelconque des revendications 5 à 10, caractérisé en ce que le matériau du tamis moléculaire est mélangé en outre à un matériau finement granulaire, inerte, hydrophobe.

12. Dispositif selon l'une quelconque des revendications 5 à 11, caractérisé en ce que le matériau du tamis moléculaire est hydrophile et/ou est imprégné avec un agent mouillant dans une proportion de 0,01 - 0,1 %.

**13.** Dispositif selon l'une quelconque des revendications 5 à 12, caractérisé en ce que le matériau du tamis moléculaire est imprégné en outre avec un tampon et/ou des sels et/ou des complexants.

**14.** Utilisation d'un matériau sec, poreux, non gonflant, granulaire, ayant des propriétés de tamis moléculaire, pour la détection d'un antigène, par réaction avec un excès d'anticorps, et séparation subséquente de l'anticorps libre et du complexe antigène-anticorps.